# EUROPEAN PATENT APPLICATION

(11) **EP 2 157 174 A1**
(43) Date of publication of application: **24.02.2010**
(21) Application number: 08162255.7
(22) Date of filing: 12.08.2008
(51) Int. Cl.: C12N 9/10, C12N 15/52

(54) **Increased production of pantothenate (vitamin B5)**

(71) Applicant: DSM IP Assets B.V., 6411 TE Heerlen (NL)
(72) Inventor: Perkins, John B., 2242 SW Wassenaar (NL); Pragai, Zoltan, 4058 Basel (CH)
(74) Representative: Seibel-Thomsen, Nadja

(57) **Abstract**

The present invention relates to newly identified *Bacillus subtilis* mutants wherein the gene encoding PanB has been modified leading to increased production of pantothenic acid (vitamin B5).

## Description

The present invention relates to newly identified *Bacillus subtilis* mutants wherein the gene encoding PanB has been modified leading to increased production of pantothenic acid (vitamin B5).

Pantothenate is a member of the B complex of vitamins and is a nutritional requirement for mammals including humans, *e*.*g*., from food sources, as a water-soluble vitamin supplement or as a feed additive. In cells, pantothenate is used primarily for the biosynthesis of coenzyme A and acyl carrier protein. These essential coenzymes function in the metabolism of acyl moieties, which form thioesters with the sulfhydryl group of the 4'-phosphopantethein portion of these molecules.

Pantothenate has been synthesized conventionally via chemical synthesis from bulk chemicals. However, the substrates required for chemical synthesis are expensive and the racemic intermediates have to be optically resolved. Accordingly, bacterial or microbial systems have been employed that produce enzymes useful in pantothenate biosynthesis processes. In particular, bioconversion processes have been evaluated as a means of favoring production of preferred isomer of pantothenic acid. Moreover, methods of direct microbial synthesis have recently been examined as a means of facilitating D-pantothenate production.

The genes involved in biosynthesis of pantothenic acid as well as methods for fermentative production of pantothenic acid are known (see *e*.*g*. WO 01/21772, WO 02/057474, WO 02/061108 or Ullman's Encyclopedia of Industrial Chemistry, 7th Edition, 2007, Chapter Vitamins).

A key enzyme in biosynthesis of pantothenic acid is PanB, an enzyme having ketopantoate hydroxymethyl transferase activity being encoded by *panB*. PanB catalyzes the conversion of α-ketoisovalerate to α-ketopantoate; α-ketopantoate in turn is converted to pantoate via the NADPH-dependent PanE reaction. It has been found that this step forms a serious bottleneck in the biosynthesis of pantothenate, i.e. is rate-limiting for pantothenate biosynthesis.

Surprisingly, it has been discovered that introducing specific mutation(s) into the *panB* gene could lead to a reduction of said bottleneck leading to increased production of pantothenate. The modified enzymes as of the present invention are less sensitive to feedback inhibition.

Thus, it is an object of the present invention to provide a pantothenate production strain wherein the *panB* bottleneck is reduced by using a modified *panB* gene. Said modified PanB enzymes are less sensitive to feedback-inhibition.

As used herein, the term "pantothenic acid" includes but is not limited to pantothenic acid, precursors and/or derivatives thereof such as salts or esters thereof, *i*.*e*. pantothenate, in particular calcium pantothenate, or the alcohol form of pantothenic acid, *i*.*e*. pantothenol or panthenol. The terms "pantothenic acid", "pantothenate" and "vitamin B5" are used interchangeably herein. Precursors/intermediates in the biosynthetic pathway of pantothenic acid which are included may be selected from *e*.*g*. pantoate, α-ketopantoate or α-ketoisovalerate.

In particular, it has now been found that by modification of the *panB*-encoded polypeptide the biosynthesis of pantothenate can be greatly improved within a microorganism, such as for example in *Bacillus,* carrying such modification and being capable of producing pantothenate.

Consequently, the invention relates to a modified or mutated polynucleotide selected from the group consisting of:
(a) polynucleotides encoding the polypeptide comprising the amino acid sequence according to SEQ ID NO:2;
(b) polynucleotides comprising the nucleotide sequence according to SEQ ID NO:1;
(c) polynucleotides comprising a nucleotide sequence obtainable by nucleic acid amplification such as polymerase chain reaction, using genomic DNA from a microorganism as a template and a primer set according to SEQ ID NO:3 and SEQ ID NO:4;
(d) polynucleotides comprising a nucleotide sequence encoding a fragment or derivative of a polypeptide encoded by a polynucleotide of any of (a) to (c) wherein in said derivative one or more amino acid residues are conservatively substituted compared to said polypeptide, and said fragment or derivative has ketopantoate hydroxymethyl transferase activity;
(e) polynucleotides the complementary strand of which hybridizes under stringent conditions to a polynucleotide as defined in any one of (a) to (d) and which encode a ketopantoate hydroxymethyl transferase polypeptide; and
(f) polynucleotides which are at least 60%, such as 70, 85, 90 or 95% identical to a polynucleotide as defined in any one of (a) to (d) and which encode a ketopantoate hydroxymethyl transferase polypeptide;
or
the complementary strand of such a polynucleotide and wherein the polynucleotide depicted under SEQ ID NO:1 comprises at least 1 mutation leading to an enzyme which is less sensitive to feedback inhibition.

In particular, the invention relates to a modified or mutated polynucleotide wherein the mutation is selected from 1 or more, i.e. 1, 2, 3, 4, substitutions on a position corresponding to a position as depicted in SEQ ID NO:2 which is selected from position 13, 35, 38, 57, 75, 76, 101, 109, 147, 226, 244, 246, 259, 272, and combinations thereof, said mutated enzymes being less sensitive to feedback inhibition.

In a preferred embodiment, the invention relates to a modified or mutated polynucleotide selected from the group consisting of:
(a) polynucleotides encoding a (modified) polypeptide comprising the amino acid sequence according to SEQ ID NO:6;
(b) polynucleotides comprising the (modified) nucleotide sequence according to SEQ ID NO:5;
(c) polynucleotides comprising a nucleotide sequence obtainable by nucleic acid amplification such as polymerase chain reaction, using genomic DNA from a microorganism as a template and a primer set according to SEQ ID NO:3 and SEQ ID NO:4;
(d) polynucleotides comprising a nucleotide sequence encoding a fragment or derivative of a polypeptide encoded by a polynucleotide of any of (a) to (c) wherein in said derivative one or more amino acid residues are conservatively substituted compared to said polypeptide, and said fragment or derivative has the activity of modified ketopantoate hydroxymethyl transferase;
(e) polynucleotides the complementary strand of which hybridizes under stringent conditions to a polynucleotide as defined in any one of (a) to (d) and which encode a ketopantoate hydroxymethyl transferase polypeptide; and
(f) polynucleotides which are at least 60%, such as 70, 85, 90 or 95% identical to a polynucleotide as defined in any one of (a) to (d) and which encode a ketopantoate hydroxymethyl transferase polypeptide;
or
the complementary strand of such a polynucleotide and wherein the sequences depicted under SEQ ID NO:5 and 6 are referred to as modified or mutated sequences and wherein said polynucleotide comprises at least one mutation leading to an enzyme which is less-sensitive to feedback inhibition compared to the corresponding wild-type polynucleotide.

In another preferred embodiment, the invention relates to a modified or mutated polynucleotide selected from the group consisting of:
(a) polynucleotides encoding a (modified) polypeptide comprising the amino acid sequence according to SEQ ID NO:8;
(b) polynucleotides comprising the (modified) nucleotide sequence according to SEQ ID NO:7;
(c) polynucleotides comprising a nucleotide sequence obtainable by nucleic acid amplification such as polymerase chain reaction, using genomic DNA from a microorganism as a template and a primer set according to SEQ ID NO:3 and SEQ ID NO:4;
(d) polynucleotides comprising a nucleotide sequence encoding a fragment or derivative of a polypeptide encoded by a polynucleotide of any of (a) to (c) wherein in said derivative one or more amino acid residues are conservatively substituted compared to said polypeptide, and said fragment or derivative has the activity of modified ketopantoate hydroxymethyl transferase;
(e) polynucleotides the complementary strand of which hybridizes under stringent conditions to a polynucleotide as defined in any one of (a) to (d) and which encode a ketopantoate hydroxymethyl transferase polypeptide; and
(f) polynucleotides which are at least 60%, such as 70, 85, 90 or 95% identical to a polynucleotide as defined in any one of (a) to (d) and which encode a ketopantoate hydroxymethyl transferase polypeptide;
or
the complementary strand of such a polynucleotide and wherein the sequences depicted under SEQ ID NO:7 and 8 are referred to as modified or mutated sequences and wherein said polynucleotide comprises at least one mutation leading to an enzyme which is less-sensitive to feedback inhibition compared to the corresponding wild-type polynucleotide.

According to a further embodiment, the invention relates to a modified or mutated polynucleotide selected from the group consisting of:
(a) polynucleotides encoding a (modified) polypeptide comprising the amino acid sequence according to SEQ ID NO:10;
(b) polynucleotides comprising the (modified) nucleotide sequence according to SEQ ID NO:9;
(c) polynucleotides comprising a nucleotide sequence obtainable by nucleic acid amplification such as polymerase chain reaction, using genomic DNA from a microorganism as a template and a primer set according to SEQ ID NO:3 and SEQ ID NO:4;
(d) polynucleotides comprising a nucleotide sequence encoding a fragment or derivative of a polypeptide encoded by a polynucleotide of any of (a) to (c) wherein in said derivative one or more amino acid residues are conservatively substituted compared to said polypeptide, and said fragment or derivative has the activity of modified ketopantoate hydroxymethyl transferase;
(e) polynucleotides the complementary strand of which hybridizes under stringent conditions to a polynucleotide as defined in any one of (a) to (d) and which encode a ketopantoate hydroxymethyl transferase polypeptide; and
(f) polynucleotides which are at least 60%, such as 70, 85, 90 or 95% identical to a polynucleotide as defined in any one of (a) to (d) and which encode a ketopantoate hydroxymethyl transferase polypeptide;
or
the complementary strand of such a polynucleotide and wherein the sequences depicted under SEQ ID NO:9 and 10 are referred to as modified or mutated sequences and wherein said polynucleotide comprises at least one mutation leading to an enzyme which is less-sensitive to feedback inhibition compared to the corresponding wild-type polynucleotide.

The terms "non-modified protein" and "wild-type *panB*-encoded protein", in particular PanB, are used interchangeably herein. Non-modified *panB*-encoded proteins or non-modified proteins may include any protein encoded by a nucleotide sequence that hybridizes preferably under highly stringent conditions to a sequence shown in SEQ ID NO:1 for which increasing the specific activity is desirable in order to relieve the *panB* bottleneck and to increase production of pantothenate in a given microorganism. These sequences are then used as starting point for designing mutants with increased activity according to the present invention. "Wild-type" in the context of the present invention may include both sequences derivable from nature as well as variants of synthetic sequences, if they can be made more active by any of the teachings of the present invention. In particular, such proteins are of prokaryotic origin, preferably bacterial origin, in particular originated from Gram positive or Gram negative bacteria, *e.g. Bacillus, Corynebacterium, Lactobacillus, Lactococcus, Streptomyces, Escherichia coli*, and *Pseudomonas.* In another preferred embodiment, the non-modified protein or gene product is derived from genera of the *Bacillus* sensu lato group, *Geobacillus, Brevibacillus* and the like (see Table 1 in Zeigler and Perkins, 2008, Practical Handbook of Microbiology", Second Edition (E. Goldman and L. Green, eds.), pp 301-329, CRC Press, Boca Raton, FL). More preferably the non-modified PanB proteins are selected from *Bacillus* species represented by the *Bacillus* sensu stricto group, in particular *Bacillus subtilis, Bacillus lentimorbus, Bacillus lentus, Bacillus anthracis, Bacillus firmus, Bacillus pantothenticus, Bacillus cereus, Bacillus circulans, Bacillus coagulans, Bacillus megaterium, Bacillus thuringiensis, Bacillus licheniformis, Bacillus amyloliquefaciens, Bacillus pumilus, Bacillus halodurans* (Zeigler and Perkins, 2008, Ibid). Most preferred, the non-modified PanB proteins are selected from *Bacillus subtilis* 168 and its strain derivatives.

The terms "modified protein" and "mutant protein", in particular PanB, are used interchangeably herein. This also applies to the terms "modified protein" and "mutant protein". A mutant/modified protein, or modified PanB protein may include any variant derivable from a given wild-type protein/PanB protein (according to the above definition) according to the teachings of the present invention and being more active (such as *e*.*g*. measurable as increase in pantothenate produced from a given substrate) than the respective wild-type enzyme. For the scope of the present invention, it is not relevant how the mutant(s) are obtained; such mutants may be obtained, *e*.*g*., by site-directed mutagenesis, saturation mutagenesis, random mutagenesis/directed evolution, chemical or UV mutagenesis of entire cells/organisms, and other methods which are known in the art. These mutants may also be generated, *e*.*g*., by designing synthetic genes, and/or produced by in vitro (cell-free) translation. For testing of specific activity, mutants may be (over-)expressed by methods known to those skilled in the art with measurement of the pantothenate production (see Examples).

A modified PanB protein of the invention may be obtained by mutating the corresponding non-modified PanB. This could be performed *e.g. via* modification of the nucleotide sequence that hybridizes preferably under highly stringent conditions to a sequence shown in SEQ ID NO:1 (*panB* gene). The non-modified PanB as isolated from *Bacillus subtilis* 168 is shown in SEQ ID NO:2.

The modified PanB protein may contain at least one mutation leading to a modified *panB-*encoded protein said at least one mutation having an impact on the biosynthesis of pantothenate which is negatively affected by the PanB bottleneck when present in a suitable microorganism. The at least one mutation may be one or more substitution, addition and/or deletion, preferably one or more amino acid substitution(s). When taking the PanB polypeptide as reference, the at least one mutation may be at least a substitution of an amino acid wherein the substitution takes place on a position corresponding to a position between amino acid 1 and 277 as depicted in SEQ ID NO:2.

Preferably, the at least one substitution is at least a replacement on a position corresponding to position 13 as depicted in SEQ ID NO:2, more preferably a substitution of S 13N by another amino acid, most preferably a replacement of S13 by N13. In a further preferred embodiment, the at least one substitution is at least a replacement on a position corresponding to position 75 as depicted in SEQ ID NO:2, more preferably a substitution of P75 by another amino acid, most preferably a replacement of P75 by Q75. In another preferred embodiment, the at least one substitution is at least a replacement on a position corresponding to position 101 as depicted in SEQ ID NO:2, more preferably a substitution of A101 by another amino acid, most preferably a replacement of A101 by V101. In another preferred embodiment, the at least one substitution is at least a replacement on a position corresponding to position 226 as depicted in SEQ ID NO:2, more preferably a substitution of T226 by another amino acid, most preferably a replacement of T226 by P226. In another preferred embodiment, the at least one substitution is at least a replacement on a position corresponding to position 244 as depicted in SEQ ID NO:2, more preferably a substitution of I244 by another amino acid, most preferably a replacement of I224 by V244. The amino acid sequence of such modified PanB is depicted in SEQ ID NO:6 as encoded by SEQ ID NO:5.

In still another preferred embodiment, the at least one substitution is at least a replacement on a position corresponding to position 35, 38, 57, 76, 109, 147, 246, 259 and/or 272 as depicted in SEQ ID NO:2, more preferably a substitution of G35, M38, G57, N76, D109, L147, G246, Q259 or G272 by another amino acid, most preferably a replacement of G by E on position 35, M by I on position 38, G by C on position 57, N by S on position 76, D by E on position 109, L by M on position 147, G by R on position 246, Q by H on position 259 or G by S on position 272.

The present invention is furthermore directed to a modified PanB polypeptide comprising at least one or more mutations, such as *e*.*g*. 1, 2, 3, or 4, listed above in combination, such as *e*.*g*. a combination of at least two mutations, in particular M38I with N76S or G57C with L147M or G246R with G272S. A combination of at least three mutations is also possible, in particular S13N with P75Q and A101V, see *e*.*g*. as depicted in SEQ ID NO:7 encoded by SEQ ID NO:8. A modified PanB may also comprise at least a combination of at least four mutations, in particular S13N with P75Q, A101V and 1244V.

The invention relates to modified PanB polypeptides and to microorganisms wherein the activity of a PanB polypeptide is enhanced and/or improved so that the yield of pantothenate is increased. This may be accomplished, for example, by modifying said PanB polypeptide in a way as described herein, *e*.*g*. by introducing a mutation in a microorganism carrying an endogenous equivalent of the *panB* gene resulting in a modified equivalent of the *panB* gene. Alternatively, an equivalent of the mutated *panB* gene as defined herein may be directly introduced into a suitable host cell suitable for producing pantothenate. The resulting modified PanB protein shows an increased activity compared to the corresponding non-modified PanB protein. A particularly useful modified PanB protein is depicted in SEQ ID NO:6 or 8 which may be encoded by SEQ ID NO:5 or 7.

The skilled person will know how to generate a mutated PanB protein. Such may be for instance accomplished by either genetically modifying the host organism in a way as described herein that it produces a mutated PanB protein which is less sensitive to feedback inhibition than the wild-type PanB/organism.

"Improved activity" or "enhanced activity" as used herein is to be understood as a relieve (totally or partial) or reduction of the PanB bottleneck in a pantothenate producing microorganism. Thus, for the purpose of the present invention, a modified PanB polypeptide with increased activity is less sensitive to feedback inhibition compared to a non-modified PanB. This can be measured (indirectly) via an increase in pantothenate production upon minimized feedback inhibition of PanB. A modified PanB is at least 5%, 10%, 25%, 50%, 75%, 100%, 200% or even more than 500% less sensitive to feedback inhibition compared to a wild-type protein. This relieve from the PanB bottleneck may be obtained via contacting the PanB protein with a specific or general enhancer of its activity.

Changes in feedback inhibition or sensitivity thereto can be measured via a change in pantothenate production, either by an in vitro enzyme assay or in vivo via shake flask experiments. Such a method is shown in Example 1. Examples of in vivo assays are described in more detail in WO 2004113510A2 (see General Methodology).

In brief, the in vitro PanB enzyme assay is performed as follows: a cell lysate containing PanB is prepared by suspending thawed frozen cell pellets of 20 ml overnight culture in 50 mM Tris and 300 mM NaCl at pH 7.5 containing 0.5-1 mg/ml lysozyme. After incubation at 4°C for 20 minutes, samples are sonicated, centrifuged for 10 minutes at 13.2 krpm at 4°C and the supernatant (i.e. the cell lysate) collected in Greiner tubes. This lysate is mixed with the reaction mixture containing PanE, α-KIV, MTHF, and NADPH. PanB activity is determined as a function or the conversion of NADPH to NADP⁺. The change of absorbance at 340 nm measures the conversion of NADPH to NADP⁺.

A nucleic acid according to the invention may be obtained by nucleic acid amplification using cDNA, mRNA or alternatively, genomic DNA, as a template and appropriate oligonucleotide primers such as the nucleotide primers according to SEQ ID NO:3 and SEQ ID NO:4 according to standard PCR amplification techniques. The nucleic acid thus amplified may be cloned into an appropriate vector and characterized by DNA sequence analysis. For obtaining mutated nucleic acid sequences, either the same or modified primers may be used, depending on the location of the mutation which should be introduced.

The template for the reaction may be cDNA obtained by reverse transcription of mRNA prepared from strains known or suspected to comprise a polynucleotide according to the invention. The PCR product may be subcloned and sequenced to ensure that the amplified sequences represent the sequences of a new nucleic acid sequence as described herein, or a functional equivalent thereof.

The PCR fragment may then be used to isolate a full length cDNA clone by a variety of known methods. For example, the amplified fragment may be labeled and used to screen a bacteriophage or cosmid cDNA library. Alternatively, the labeled fragment may be used to screen a genomic library.

Accordingly, the invention relates to polynucleotides comprising a nucleotide sequence obtainable by nucleic acid amplification such as polymerase chain reaction, using DNA such as genomic DNA from a microorganism as a template and a primer set according to SEQ ID NO:3 and SEQ ID NO:4.

The invention also relates to polynucleotides comprising a nucleotide sequence encoding a fragment or derivative of a polypeptide encoded by a polynucleotide as described herein wherein in said derivative one or more amino acid residues are conservatively substituted compared to said polypeptide, and said fragment or derivative has the activity or sensitivity to feedback inhibition of a wild-type and modified PanB polypeptide, respectively.

The invention also relates to polynucleotides the complementary strand of which hybridizes under stringent conditions to a polynucleotide as defined herein and which encode a wild-type and modified PanB polypeptide, respectively.

The invention also relates to polynucleotides which are at least 60% identical to a polynucleotide as defined herein and which encode a PanB polypeptide; and the invention also relates to polynucleotides being the complementary strand of a polynucleotide as defined herein above.

The invention also relates to primers, probes and fragments that may be used to amplify or detect a DNA according to the invention and to identify related species or families of microorganisms also carrying such genes.

The present invention also relates to vectors which include polynucleotides of the invention and microorganisms which are genetically engineered with the polynucleotides or said vectors.

The invention also relates to processes for producing microorganisms capable of expressing a polypeptide encoded by the above defined polynucleotide and a polypeptide encoded by a polynucleotide as defined above, in particular a modified polypeptide as defined herein.

Suitable host cells include cells of microorganisms capable of producing pantothenate, *e*.*g*., converting a given carbon source into pantothenate and which carry either a non-modified *panB* gene or equivalent or homologue thereof (which is then mutated in such a way that it leads to an increase in pantothenate production as described herein) or into which a modified version of said *panB* gene or equivalent thereof is introduced. Suitable microorganisms carrying such a non-modified gene or equivalent thereof may be selected from bacteria, such as Gram positive or Gram negative bacteria, either as wild-type strains, mutant strains derived by classic mutagenesis and selection methods or as recombinant strains. Examples of such bacteria may be, *e.g., Bacillus, Corynebacterium, Lactobacillus, Lactococcus, Streptomyces, Escherichia coli*, and *Pseudomonas.* In another preferred embodiment, the host cell is selected from *Geobacillus* or *Brevibacillus* of the *Bacillus* sensu lato group (see Table 1 in Zeigler and Perkins, 2008, Practical Handbook of Microbiology", Second Edition (E. Goldman and L. Green, eds.), pp 301-329, CRC Press, Boca Raton, FL). Furthermore, a suitable host cell may be selected from *Bacillus* species represented by the *Bacillus* sensu stricto group, in particular *Bacillus subtilis, Bacillus lentimorbus, Bacillus lentus, Bacillus anthracis, Bacillus firmus, Bacillus pantothenticus, Bacillus cereus, Bacillus circulans, Bacillus coagulans, Bacillus megaterium, Bacillus thuringiensis, Bacillus licheniformis, Bacillus amyloliquefaciens, Bacillus pumilus, Bacillus halodurans* (Zeigler and Perkins, 2008, Ibid). Most preferred, the microorganism is selected from *Bacillus subtilis* 168 and derivatives thereof.

All microorganisms which can be used for the present invention may be publicly available from different sources, *e*.*g*., Deutsche Sammlung von Mikroorganismen und Zellkulturen (DSMZ), Inhoffenstr. 7B, D-38124 Braunschweig, Germany, American Type Culture Collection (ATCC), P.O. Box 1549, Manassas, VA 20108 USA or Culture Collection Division, NITE Biological Resource Center, 2-5-8, Kazusakamatari, Kisarazu-shi, Chiba, 292-0818, Japan (formerly: Institute for Fermentation, Osaka (IFO), 17-85, Juso-honmachi 2-chome,Yodogawa-ku, Osaka 532-8686, Japan).

In connection with the present invention it is understood that the above-mentioned microorganisms also include synonyms or basonyms of such species having the same physiological properties, as defined by the International Code of Nomenclature of Prokaryotes. The nomenclature of the microorganisms as used herein is the one officially accepted (at the filing date of the priority application) by the International Committee on Systematics of Prokaryotes and the Bacteriology and Applied Microbiology Division of the International Union of Microbiological Societies, and published by its official publication vehicle International Journal of Systematic and Evolutionary Microbiology (IJSEM).

The present invention is directed to modified microorganisms, wherein said modification leads to an increased yield, production and/or efficiency of pantothenate from substrates like *e*.*g*. α-ketoisovalerate. This may be performed by increasing the activity of the *panB* gene as described herein. In addition, a microorganism as of the present invention may carry further modifications either on the DNA or protein level (see above), as long as such modification has a direct impact on the yield, production and/or efficiency of pantothenate production.

Thus, in one embodiment the present invention is directed to a microorganism carrying a modified PanB as described herein and additionally modification, such as *e*.*g*. via overexpression of the respective gene, in the PanE protein which leads to an increase in pantothenate production which is at least 5%, 10%, 25%, 30%, 40%, 50%, 75%, 100%, 200% or even more than 500%, compared to a genetically altered microorganism carrying only a modification in the PanB protein. This may be furthermore increased by replacing the natural promoter of the pantothenate operon by a strong (constitutive or inducible) promoter such as *e*.*g*. SPO1-15 (P15) or Pₐᵣₐ (Lee, G. and J. Pero. 1981. J. Mod. Biol. 152:247-265; Noqueira et al., 1997, Microbiology 143: 957-969). A protocol how these mutants may be obtained and which can be applied accordingly is given in WO 2004113510A2 (see Example 1). The introduction of a strong promoter results in a further increase in pantothenate production which is at least 5%, 10%, 25%, 30%, 40%, 50%, 75%, 100%, 200% or even more than 500%, compared to a microorganism wherein the PanB and PanE polypeptides are modified but the natural promoter is still present.

Also known in the art are methods of increasing the activity of a given protein by contacting the respective PanB protein with specific enhancers or other substances that specifically interact with said PanB protein. In order to identify such specific enhancers, the PanB protein may be expressed and tested for activity in the presence of compounds suspected to enhance the activity of the given protein(s). The activity of such a PanB protein may also be increased by stabilizing the messenger RNA encoding it. Such methods are also known in the art, see for example, in Sambrook et al., 1989, Molecular Cloning, A Laboratory Manual, Cold Spring Harbor Press, N. Y.; and Ausubel et al. (eds.), 1995, Current Protocols in Molecular Biology, (John Wiley & Sons, N.Y.) or WO 2007065602A1 (see in particular Examples 1 to 8).

In accordance with a further object of the present invention there is provided the use of a polynucleotide as defined above or a microorganism which is genetically engineered with such polynucleotides in the pantothenate production.

The invention also relates to processes for the expression of (modified) genes in a microorganism, to processes for the production of polypeptides as defined above in a microorganism and to processes for the production of microorganisms capable of producing pantothenate. All these processes may comprise the step of altering a microorganism, wherein "altering" as used herein encompasses the process for "genetically altering" or "altering the composition of the cell culture media and/or methods used for culturing" in such a way that the yield and/or productivity of pantothenate can be improved compared to the wild-type microorganism. The term "altering" also includes the generation of modified polynucleotides and/or polypeptides as described herein, in particular modification of the *panB* gene/polypeptide. As used herein, "improved yield of pantothenate" means an increase of at least 5%, 10%, 25%, 30%, 40%, 50%, 75%, 100%, 200% or even more than 500%, compared to a wild-type microorganism, *i*.*e*. a microorganism which is not genetically altered.

The term "genetically engineered" or "genetically altered" means the scientific alteration of the structure of genetic material in a living organism. It involves the production and use of recombinant DNA. More in particular it is used to delineate the genetically engineered or modified organism from the naturally occurring organism. Genetic engineering may be done by a number of techniques known in the art, such as *e*.*g*. gene replacement, gene amplification, gene disruption, transfection, transformation using plasmids, viruses, or other vectors. A genetically modified organism, *e*.*g*. genetically modified microorganism, is also often referred to as a recombinant organism, *e*.*g*. recombinant microorganism.

According to the invention a genetically engineered/recombinantly produced host cell (also referred to as recombinant cell or transformed cell) carrying such a modified polynucleotide wherein the function of the linked protein is significantly modified in comparison to a wild-type cell such that the yield, production and/or efficiency of production of pantothenate is improved. The host cell may be selected from a microorganism capable of producing pantothenate from a given carbon source, in particular *Bacillus subtilis.*

A "transformed cell" or "recombinant cell" is a cell into which (or into an ancestor of which) has been introduced, by means of recombinant DNA techniques, a nucleic acid according to the invention, or wherein the sensitivity of (endogenous) PanB protein for feedback inhibition has been reduced by modification of the same as defined herein. Host cells which may be useful for performing the present invention and which do not naturally carry such a gene as *e*.*g*. the gene encoding (non-modified) PanB but which are genetically modified by introduction of a mutated gene as defined herein include, but are not limited to, strains from the genera *Bacillus, Corynebacterium, Lactobacillus, Lactococcus, Streptomyces, Escherichia coli*, and *Pseudomonas.* In another preferred embodiment, the host cell is selected from *Geobacillus* or *Brevibacillus* of the *Bacillus* sensu lato group (see Table 1 in Zeigler and Perkins, 2008, Practical Handbook of Microbiology", Second Edition (E. Goldman and L. Green, eds.), pp 301-329, CRC Press, Boca Raton, FL). Furthermore, a suitable host cell may be selected from *Bacillus* species represented by the *Bacillus* sensu stricto group, in particular *Bacillus subtilis, Bacillus lentimorbus, Bacillus lentus, Bacillus anthracis, Bacillus firmus, Bacillus pantothenticus, Bacillus cereus, Bacillus circulans, Bacillus coagulans, Bacillus megaterium, Bacillus thuringiensis, Bacillus licheniformis, Bacillus amyloliquefaciens, Bacillus pumilus, Bacillus halodurans* (Zeigler and Perkins, 2008, Ibid). Most preferred, the microorganism is selected from *Bacillus subtilis* 168 and derivatives thereof.

Embodiments of the invention include both the genetically altering of a microorganism carrying an endogenous gene encoding (non-modified) PanB protein or an equivalent thereof such that said (modified) gene product is less sensitive to feedback inhibition and they also include the introduction of said modified polynucleotide or equivalent thereof as described above into a suitable host organism not naturally carrying such a gene and being capable of producing pantothenate and furthermore being capable of expressing said introduced (modified) gene.

The invention also relates to a polynucleotide encoding at least a biologically active fragment or derivative of the polypeptides as described herein, in particular a PanB polypeptide as shown in SEQ ID NO:2 or a modified PanB polypeptide as shown in SEQ ID NO:6 or 8.

As used herein, "biologically active fragment or derivative" means a polypeptide which retains essentially the same biological function or activity as the polypeptide shown in SEQ ID NO:2, 6 or 8. Examples of biological activity may for instance be enzymatic activity, signaling activity or antibody reactivity. The term "same biological function" or "functional equivalent" as used herein means that the protein has essentially the same biological activity, *e*.*g*. enzymatic, signaling or antibody reactivity, as a polypeptide shown in SEQ ID NO:2, 6 or 8.

The biological, enzymatic or other activity of PanB proteins, in particular the wild-type and modified PanB protein, respectively, can be measured by methods well known to a skilled person, such as, for example, by an assay described herein.

The polypeptides and polynucleotides of the present invention are preferably provided in an isolated form, and preferably are purified to homogeneity.

The term "isolated" means that the material is removed from its original environment (*e*.*g*., the natural environment if it is naturally occurring). For example, a naturally-occurring polynucleotide or polypeptide present in a living microorganism is not isolated, but the same polynucleotide or polypeptide, separated from some or all of the coexisting materials in the natural system, is isolated. Such polynucleotides could be part of a vector and/or such polynucleotides or polypeptides could be part of a composition and still be isolated in that such vector or composition is not part of its natural environment.

An isolated polynucleotide or nucleic acid as used herein may be a DNA or RNA that is not immediately contiguous with both of the coding sequences with which it is immediately contiguous (one on the 5'-end and one on the 3'-end) in the naturally occurring genome of the organism from which it is derived. Thus, in one embodiment, a nucleic acid includes some or all of the 5'-non-coding (*e*.*g*., promoter) sequences that are immediately contiguous to the coding sequence. The term "isolated polynucleotide" therefore includes, for example, a recombinant DNA that is incorporated into a vector, into an autonomously replicating plasmid or virus, or into the genomic DNA of a prokaryote or eukaryote, or which exists as a separate molecule (*e*.*g*., a cDNA or a genomic DNA fragment produced by PCR or restriction endonuclease treatment) independent of other sequences. It also includes a recombinant DNA that is part of a hybrid gene encoding an additional polypeptide that is substantially free of cellular material, viral material, or culture medium (when produced by recombinant DNA techniques), or chemical precursors or other chemicals (when chemically synthesized). Moreover, an "isolated nucleic acid fragment" is a nucleic acid fragment that is not naturally occurring as a fragment and would not be found in the natural state.

As used herein, the terms "polynucleotide", "gene" and "recombinant gene" refer to nucleic acid molecules which may be isolated from chromosomal DNA, which include an open reading frame encoding a protein, *e.g. B. subtilis* PanB protein. A polynucleotide may include a polynucleotide sequence as shown in SEQ ID NO:1, 5, 7 or fragments thereof and regions upstream and downstream of the gene sequences which may include, for example, promoter regions, regulator regions and terminator regions important for the appropriate expression and stabilization of the polypeptide derived thereof.

A gene may include coding sequences, non-coding sequences such as for instance untranslated sequences located at the 3'- and 5'-ends of the coding region of a gene, and regulatory sequences. Moreover, a gene refers to an isolated nucleic acid molecule as defined herein. It is furthermore appreciated by the skilled person that DNA sequence polymorphisms that lead to changes in the amino acid sequences of PanB proteins may exist within a population. Such genetic polymorphism in the *panB* gene may exist among individuals within a population due to natural variation or in cells from different populations. Such natural variations can typically result in 1-5% variance in the nucleotide sequence of the *panB* gene. Any and all such nucleotide variations and the resulting amino acid polymorphism in PanB are the result of natural variation and that do not alter the functional activity of the *panB* protein are intended to be within the scope of the invention.

As used herein, the terms "polynucleotide" or "nucleic acid molecule" are intended to include DNA molecules (*e*.*g*., cDNA or genomic DNA) and RNA molecules (*e*.*g*., mRNA) and analogs of the DNA or RNA generated using nucleotide analogs. The nucleic acid molecule may be single-stranded or double-stranded, but preferably is double-stranded DNA. The nucleic acid may be synthesized using oligonucleotide analogs or derivatives (*e*.*g*., inosine or phosphorothioate nucleotides). Such oligonucleotides may be used, for example, to prepare nucleic acids that have altered base-pairing abilities or increased resistance to nucleases.

The sequence information as provided herein should not be so narrowly construed as to require inclusion of erroneously identified bases. The specific sequences disclosed herein may be readily used to isolate the complete gene from a recombinant or non-recombinant microorganism capable of producing pantothenate.

Unless otherwise indicated, all nucleotide sequences determined by sequencing a DNA molecule herein were determined using an automated DNA sequencer and all amino acid sequences of polypeptides encoded by DNA molecules determined herein were predicted by translation of a DNA sequence determined as above. Therefore, as is known in the art for any DNA sequence determined by this automated approach, any nucleotide sequence determined herein may contain some errors. Nucleotide sequences determined by automation are typically at least about 90% identical, more typically at least about 95% to at least about 99.9% identical to the actual nucleotide sequence of the sequenced DNA molecule. The actual sequence may be more precisely determined by other approaches including manual DNA sequencing methods well known in the art. As is also known in the art, a single insertion or deletion in a determined nucleotide sequence compared to the actual sequence will cause a frame shift in translation of the nucleotide sequence such that the predicted amino acid sequence encoded by a determined nucleotide sequence will be completely different from the amino acid sequence actually encoded by the sequenced DNA molecule, beginning at the point of such an insertion or deletion.

The person skilled in the art is capable of identifying such erroneously identified bases and knows how to correct for such errors.

A nucleic acid molecule according to the invention may comprise only a portion or a fragment of the nucleic acid sequence provided by the present invention, such as for instance the sequence shown in SEQ ID NO:1, for example a fragment which may be used as a probe or primer such as for instance SEQ ID NO:3 or SEQ ID NO:4 or a fragment encoding a portion of a protein according to the invention. The nucleotide sequence determined from the cloning of the *panB* gene allows for the generation of probes and primers designed for use in identifying and/or cloning other *panB* family members, as well as *panB* homologues from other species. The probe/primer typically comprises substantially purified oligonucleotides which typically comprises a region of nucleotide sequence that hybridizes preferably under highly stringent conditions to at least about 12 or 15, preferably about 18 or 20, more preferably about 22 or 25, even more preferably about 30, 35, 40, 45, 50, 55, 60, 65, or 75 or more consecutive nucleotides of a nucleotide sequence shown in SEQ ID NO:1 or a fragment or derivative thereof. The same may be applicable with respect to SEQ ID NO:5 or 7 or the mutated *panB* gene and homologs thereof.

A nucleic acid molecule encompassing all or a portion of the nucleic acid sequence of SEQ ID NO:1, 5 or 7 may be also isolated by the polymerase chain reaction (PCR) using synthetic oligonucleotide primers designed based upon the sequence information contained herein.

A nucleic acid of the invention may be amplified using cDNA, mRNA or alternatively, genomic DNA, as a template and appropriate oligonucleotide primers according to standard PCR amplification techniques. The nucleic acid thus amplified may be cloned into an appropriate vector and characterized by DNA sequence analysis.

Fragments of a polynucleotide according to the invention may also comprise polynucleotides not encoding functional polypeptides. Such polynucleotides may function as probes or primers for a PCR reaction.

Nucleic acids according to the invention irrespective of whether they encode functional or non-functional polypeptides, may be used as hybridization probes or polymerase chain reaction (PCR) primers. Uses of the nucleic acid molecules of the present invention that do not encode a polypeptide having a PanB activity include, inter alia, (1) isolating the gene encoding the protein of the present invention, or allelic variants thereof from a cDNA library, *e*.*g*., from other organisms than *Bacillus subtilis* and (2) Northern blot analysis for detecting expression of mRNA of said protein in specific cells or (3) use in enhancing and/or improving the function or activity of homologous *panB* genes in said other organisms.

Probes based on the nucleotide sequences provided herein may be used to detect transcripts or genomic sequences encoding the same or homologous proteins for instance in other organisms. Nucleic acid molecules corresponding to natural variants and non-B. *subtilis* homologues of the *B. subtilis panB* DNA which are also embraced by the present invention may be isolated based on their homology to the *B. subtilis panB* nucleic acid disclosed herein using the *B. subtilis* DNA, or a portion thereof, as a hybridization probe according to standard hybridization techniques, preferably under highly stringent hybridization conditions. This applies both to the wild-type and modified *panB* DNA as described herein.

In preferred embodiments, the probe further comprises a label group attached thereto, *e*.*g*., the label group can be a radioisotope, a fluorescent compound, an enzyme, or an enzyme cofactor.

Homologous gene sequences may be isolated, for example, by performing PCR using two degenerate oligonucleotide primer pools designed on the basis of nucleotide sequences as taught herein.

The template for the reaction may be cDNA obtained by reverse transcription of mRNA prepared from strains known or suspected to express a polynucleotide according to the invention. The PCR product may be subcloned and sequenced to ensure that the amplified sequences represent the sequences of a new nucleic acid sequence as described herein, or a functional equivalent thereof.

The PCR fragment may then be used to isolate a full length cDNA clone by a variety of known methods. For example, the amplified fragment may be labeled and used to screen a bacteriophage or cosmid cDNA library. Alternatively, the labeled fragment may be used to screen a genomic library.

PCR technology can also be used to isolate full-length cDNA sequences from other organisms. For example, RNA may be isolated, following standard procedures, from an appropriate cellular or tissue source. A reverse transcription reaction may be performed on the RNA using an oligonucleotide primer specific for the most 5'-end of the amplified fragment for the priming of first strand synthesis.

The resulting RNA/DNA hybrid may then be "tailed" (*e*.*g*., with guanines) using a standard terminal transferase reaction, the hybrid may be digested with RNaseH, and second strand synthesis may then be primed (*e*.*g*., with a poly-C primer). Thus, cDNA sequences upstream of the amplified fragment may easily be isolated. For a review of useful cloning strategies, see *e*.*g*., Sambrook et al., supra; and Ausubel et al., supra.

Also, nucleic acids encoding other *panB* family members, which thus have a nucleotide sequence that differs from a nucleotide sequence according to SEQ ID NO:1, are within the scope of the invention. Moreover, nucleic acids encoding PanB proteins from different species which thus may have a nucleotide sequence which differs from a nucleotide sequence shown in SEQ ID NO:1 are within the scope of the invention. All these sequences may then be used for modification as defined herein.

The invention also relates to an isolated polynucleotide hybridizable under stringent conditions, preferably under highly stringent conditions, to a polynucleotide as of the present invention, such as for instance a polynucleotide shown in SEQ ID NO:1, 5 or 7. Advantageously, such polynucleotide may be obtained from a microorganism capable of biosynthesis of pantothenate, in particular *B. subtilis*.

As used herein, the term "hybridizing" is intended to describe conditions for hybridization and washing under which nucleotide sequences at least about 50%, at least about 60%, at least about 70%, more preferably at least about 80%, even more preferably at least about 85% to 90%, most preferably at least 95% homologous to each other typically remain hybridized to each other.

In one embodiment, a nucleic acid of the invention is at least 40%, 45%, 50%, 55%, 60%, 65%, 70%, 75%, 80%, 85%, 90%, 91%, 92%, 93%, 94%, 95%, 96%, 97%, 98%, 99%, or more homologous to a nucleic acid sequence shown in SEQ ID NO:1, 5, 7 or the complements thereof.

A preferred, non-limiting example of stringent hybridization conditions are hybridization in 6x sodium chloride/sodium citrate (SSC) at about 45°C, followed by one or more washes in 1x SSC, 0.1% SDS at 50°C, preferably at 55°C, more preferably at 60°C and even more preferably at 65°C.

Highly stringent conditions include incubations at 42°C for a period of several days, such as 2-4 days, using a labeled DNA probe, such as a digoxygenin (DIG)-labeled DNA probe, followed by one or more washes in 2x SSC, 0.1 % SDS at room temperature and one or more washes in 0.5x SSC, 0.1% SDS or 0.1x SSC, 0.1% SDS at 65-68°C. In particular, highly stringent conditions include, for example, 2 h to 4 days incubation at 42°C using a DIG-labeled DNA probe (prepared by *e*.*g*. using a DIG labeling system; Roche Diagnostics GmbH, 68298 Mannheim, Germany) in a solution such as DigEasyHyb solution (Roche Diagnostics GmbH) with or without 100 µg/ml salmon sperm DNA, or a solution comprising 50% formamide, 5x SSC (150 mM NaCl, 15 mM trisodium citrate), 0.02% sodium dodecyl sulfate, 0.1 % N-lauroylsarcosine, and 2% blocking reagent (Roche Diagnostics GmbH), followed by washing the filters twice for 5 to 15 minutes in 2x SSC and 0.1% SDS at room temperature and then washing twice for 15-30 minutes in 0.5x SSC and 0.1% SDS or 0.1x SSC and 0.1% SDS at 65-68°C.

Preferably, an isolated nucleic acid molecule of the invention that hybridizes under preferably highly stringent conditions to a nucleotide sequence of the invention corresponds to a naturally-occurring nucleic acid molecule. As used herein, a "naturally-occurring" nucleic acid molecule refers to an RNA or DNA molecule having a nucleotide sequence that occurs in nature (*e*.*g*., encodes a natural protein). In one embodiment, the nucleic acid encodes a natural *B. subtilis* protein.

The skilled artisan will know which conditions to apply for stringent and highly stringent hybridization conditions. Additional guidance regarding such conditions is readily available in the art, for example, in Sambrook et al., 1989, Molecular Cloning, A Laboratory Manual, Cold Spring Harbor Press, N. Y.; and Ausubel et al. (eds. ), 1995, Current Protocols in Molecular Biology, (John Wiley & Sons, N. Y.). Of course, a polynucleotide which hybridizes only to a poly (A) sequence (such as the 3'-terminal poly (A) tract of mRNAs), or to a complementary stretch of T (or U) residues, would not be included in a polynucleotide of the invention used to specifically hybridize to a portion of a nucleic acid of the invention, since such a polynucleotide would hybridize to any nucleic acid molecule containing a poly (A) stretch or the complement thereof (*e*.*g*., practically any double-stranded cDNA clone).

For example, polynucleotide strains may be screened for homologous polynucleotides by Southern and/or Northern blot analysis. Upon detection of transcripts homologous to polynucleotides according to the invention, DNA libraries may be constructed from RNA isolated from the appropriate strain, utilizing standard techniques well known to those of skill in the art. Alternatively, a total genomic DNA library may be screened using a probe hybridizable to a polynucleotide according to the invention.

A nucleic acid molecule of the present invention, such as for instance a nucleic acid molecule shown in SEQ ID NO:1, 5, 7 or fragments or derivatives thereof, may be isolated using standard molecular biology techniques and the sequence information provided herein. For example, using all or portion of the nucleic acid sequence shown in SEQ ID NO:1, 5 or 7 as a hybridization probe, nucleic acid molecules according to the invention may be isolated using standard hybridization and cloning techniques (*e*.*g*., as described in Sambrook, J., Fritsh, E. F. , and Maniatis, T. Molecular Cloning : A Laboratory Manual. 2nd, ed. , Cold Spring Harbor Laboratory, Cold Spring Harbor Laboratory Press, Cold Spring Harbor, NY, 1989).

Furthermore, oligonucleotides corresponding to or hybridizable to nucleotide sequences according to the invention may be prepared by standard synthetic techniques, *e*.*g*., using an automated DNA synthesizer.

The terms "homology" or "percent identity" are used interchangeably herein. For the purpose of this invention, it is defined here that in order to determine the percent identity of two amino acid sequences or of two nucleic acid sequences, the sequences are aligned for optimal comparison purposes (*e*.*g*., gaps may be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (*i*.*e*., % identity = number of identical positions/total number of positions (*i*.*e*., overlapping positions) x 100). Preferably, the two sequences are the same length.

The skilled person will be aware of the fact that several different computer programs are available to determine the homology between two sequences. For instance, a comparison of sequences and determination of percent identity between two sequences may be accomplished using a mathematical algorithm. In a preferred embodiment, the percent identity between two amino acid sequences is determined using the Needleman and Wunsch (J. Mol. Biol. (48): 444-453 (1970)) algorithm which has been incorporated into the GAP program in the GCG software package (available at http://www.accelrys.com), using either a Blossom 62 matrix or a PAM250 matrix, and a gap weight of 16, 14, 12, 10, 8, 6 or 4 and a length weight of 1, 2, 3, 4, 5 or 6. The skilled person will appreciate that all these different parameters will yield slightly different results but that the overall percentage identity of two sequences is not significantly altered when using different algorithms.

In yet another embodiment, the percent identity between two nucleotide sequences is determined using the GAP program in the GCG software package (available at http://www.accelrys.com), using a NWSgapdna.CMP matrix and a gap weight of 40, 50, 60, 70 or 80 and a length weight of 1, 2, 3, 4, 5 or 6. In another embodiment, the percent identity between two amino acid or nucleotide sequences is determined using the algorithm of E. Meyers and W. Miller (CABIOS, 4: 11-17 (1989) which has been incorporated into the ALIGN program (version 2.0) (available at http://vega.igh.cnrs.fr/bin/align-guess.cgi) using a PAM120 weight residue table, a gap length penalty of 12 and a gap penalty of 4.

The nucleic acid and protein sequences of the present invention may further be used as a "query sequence" to perform a search against public databases to, for example, identify other family members or related sequences. Such searches may be performed using the BLASTN and BLASTX programs (version 2.0) of Altschul, et al. (1990) J. Mol. Biol. 215:403-10. BLAST nucleotide searches may be performed with the BLASTN program, score = 100, word length = 12 to obtain nucleotide sequences homologous to the nucleic acid molecules of the present invention. BLAST protein searches may be performed with the BLASTX program, score = 50, word length = 3 to obtain amino acid sequences homologous to the protein molecules of the present invention. To obtain gapped alignments for comparison purposes, Gapped BLAST may be utilized as described in Altschul et al., (1997) Nucleic Acids Res. 25 (17): 3389-3402. When utilizing BLAST and Gapped BLAST programs, the default parameters of the respective programs (*e*.*g*., BLASTX and BLASTN) may be used. See http://www.ncbi.nlm.nih.gov.

In another preferred embodiment, an isolated nucleic acid molecule of the invention comprises a nucleic acid molecule which is the complement of a nucleotide sequence as of the present invention, such as for instance the sequence shown in SEQ ID NO:1, 5 or 7. A nucleic acid molecule, which is complementary to a nucleotide sequence disclosed herein, is one that is sufficiently complementary to a nucleotide sequence shown in SEQ ID NO:1, 5 or 7 such that it may hybridize to said nucleotide sequence thereby forming a stable duplex.

Another aspect of the invention pertains to vectors, containing a nucleic acid encoding a protein according to the invention or a functional equivalent or portion thereof. As used herein, the term "vector" refers to a nucleic acid molecule capable of transporting another nucleic acid to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (*e*.*g*., bacterial vectors having a bacterial origin of replication). Other vectors are integrated into the genome of a host cell upon introduction into the host cell, and thereby are replicated along with the host genome.

Moreover, certain vectors are capable of directing the expression of genes to which they are operatively linked. Such vectors are referred to herein as "expression vectors". In general, expression vectors of utility in recombinant DNA techniques are often in the form of plasmids. The terms "plasmid" and "vector" can be used interchangeably herein as the plasmid is the most commonly used form of vector. However, the invention is intended to include such other forms of expression vectors, such as viral vectors (*e*.*g*., replication defective retroviruses, adenoviruses and adeno-associated viruses), which serve equivalent functions.

The recombinant vectors of the invention comprise a nucleic acid of the invention in a form suitable for expression of the nucleic acid in a host cell, which means that the recombinant expression vector includes one or more regulatory sequences, selected on the basis of the host cells to be used for expression, which is operatively linked to the nucleic acid sequence to be expressed. Within a recombinant expression vector, "operatively linked" is intended to mean that the nucleotide sequence of interest is linked to the regulatory sequence(s) in a manner which allows for expression of the nucleotide sequence (*e*.*g*., in an *in vitro* transcription/translation system or in a host cell when the vector is introduced into the host cell). The term "regulatory sequence" is intended to include promoters, enhancers and other expression control elements (*e*.*g*., attenuator). Such regulatory sequences are described, for example, in Goeddel; Gene Expression Technology: Methods in Enzymology 185, Academic Press, San Diego, CA (1990). Regulatory sequences include those which direct constitutive or inducible expression of a nucleotide sequence in many types of host cells and those which direct expression of the nucleotide sequence only in a certain host cell (*e*.*g*. tissue-specific regulatory sequences). It will be appreciated by those skilled in the art that the design of the expression vector can depend on such factors as the choice of the host cell to be transformed, the level of expression of protein desired, etc. The expression vectors of the invention may be introduced into host cells to thereby produce proteins or peptides, encoded by nucleic acids as described herein, including, but not limited to, mutant proteins, fragments thereof, variants or functional equivalents thereof, and fusion proteins, encoded by a nucleic acid as described herein, *e*.*g*., *panB*-encoded proteins, mutant forms of PanB proteins, fusion proteins and the like including further *panB*-encoded proteins mentioned herein.

The recombinant expression vectors of the invention may be designed for expression of (modified) PanB proteins in a suitable microorganism. For example, a protein according to the invention may be expressed in bacterial cells such as strains belonging to the genera *Bacillus.* Expression vectors useful in the present invention include chromosomal-, episomal- and virus-derived vectors *e*.*g*., vectors derived from bacterial plasmids, bacteriophage, and vectors derived from combinations thereof, such as those derived from plasmid, transposon and bacteriophage genetic elements, such as *e*.*g*. cosmids and phagemids.

The DNA insert may be operatively linked to an appropriate promoter, which may be either a constitutive or inducible promoter, such as *e*.*g*. P15. The skilled person will know how to select suitable promoters. The expression constructs may contain sites for transcription initiation, termination, and, in the transcribed region, a ribosome binding site for translation. The coding portion of the mature transcripts expressed by the constructs may preferably include an initiation codon at the beginning and a termination codon appropriately positioned at the end of the polypeptide to be translated.

Vector DNA may be introduced into suitable host cells via conventional transformation or transfection techniques. As used herein, the terms "transformation", "transconjugation" and "transfection" are intended to refer to a variety of art-recognized techniques for introducing foreign nucleic acid (*e*.*g*., DNA) into a host cell, including calcium phosphate or calcium chloride co-precipitation, DEAE-dextran-mediated transfection, transduction, infection, lipofection, cationic lipidmediated transfection or electroporation. Suitable methods for transforming or transfecting host cells may be found in Sambrook, et al. (supra), Davis et al., Basic Methods in Molecular Biology (1986) and other laboratory manuals.

In order to identify and select cells which have integrated the foreign DNA into their genome, a gene that encodes a selectable marker (*e*.*g*., resistance to antibiotics) is generally introduced into the host cells along with the gene of interest. Preferred selectable markers include those that confer resistance to drugs, such as kanamycin, tetracycline, ampicillin and streptomycin. A nucleic acid encoding a selectable marker is preferably introduced into a host cell on the same vector as that encoding a protein according to the invention or can be introduced on a separate vector such as, for example, a suicide vector, which cannot replicate in the host cells. Cells stably transfected with the introduced nucleic acid can be identified by drug selection (*e*.*g*., cells that have incorporated the selectable marker gene will survive, while the other cells die).

The invention provides also an isolated polypeptide having the amino acid sequence shown in SEQ ID NO:6 and 8, respectively, or an amino acid sequence obtainable by expressing a polynucleotide of the present invention, such as for instance a polynucleotide sequence shown in SEQ ID NO:5 and 7, respectively, in an appropriate host.

Polypeptides according to the invention may contain only conservative substitutions of one or more amino acids in the amino acid sequence represented by SEQ ID NO:2, 6, 8 or substitutions, insertions or deletions of non-essential amino acids. Accordingly, a non-essential amino acid is a residue that may be altered in the amino acid sequences shown in SEQ ID NO:2, 6 or 8 without substantially altering the biological function. For example, amino acid residues that are conserved among the proteins of the present invention, are predicted to be particularly amenable to alteration. Furthermore, amino acids conserved among the proteins according to the present invention and other PanB proteins are not likely to be amenable to alteration.

In one embodiment, the present invention is related to a microorganism which contains a mutated PanB polypeptide as described above.

The term "conservative substitution" is intended to mean that a substitution in which the amino acid residue is replaced with an amino acid residue having a similar side chain. These families are known in the art and include amino acids with basic side chains (*e*.*g*., lysine, arginine and histidine), acidic side chains (*e*.*g*., aspartic acid, glutamic acid), uncharged polar side chains (*e*.*g*., glycine, asparagine, glutamine, serine, threonine, tyrosine, cysteine), non-polar side chains (*e*.*g*., alanine, valine, leucine, isoleucine, proline, phenylalanine, methionine, tryptophan), beta-branched side chains (*e*.*g*., threonine, valine, isoleucine) and aromatic side chains (*e*.*g*., tyrosine, phenylalanine, tryptophan, histidine).

As mentioned above, the polynucleotides of the invention may be utilized in the genetic engineering of a suitable host cell to make it better and more efficient in the fermentation process for pantothenate.

The alteration in the genome of the microorganism may be obtained *e*.*g*. by replacing through a single or double crossover recombination a wild-type DNA sequence by a DNA sequence containing the alteration. For convenient selection of transformants of the microorganism with the alteration in its genome the alteration may, *e*.*g*. be a DNA sequence encoding an antibiotic resistance marker or a gene complementing a possible auxotrophy of the microorganism. Mutations include, but are not limited to, deletion-insertion mutations.

An alteration in the genome of the microorganism leading to a more functional polypeptide may also be obtained by randomly mutagenizing the genome of the microorganism using *e*.*g*. chemical mutagens, radiation or transposons and selecting or screening for mutants which are better or more efficient producers of one or more fermentation products. Standard methods for screening and selection are known to the skilled person.

The aforementioned mutagenesis strategies for PanB proteins may result in increased yields of pantothenate. This list is not meant to be limiting; variations on these mutagenesis strategies will be readily apparent to one of ordinary skill in the art. By these mechanisms, the nucleic acid and protein molecules of the invention may be utilized to generate microorganisms such as *Bacillus subtilis* or related strains of bacteria expressing mutated PanB nucleic acid and protein molecules such that the yield, productivity, and/or efficiency of production of pantothenate is improved.

The nucleic acid molecules, polypeptides, vectors, primers, and recombinant microorganisms described herein may be used in one or more of the following methods: identification of *Bacillus subtilis* and related organisms; mapping of genomes of organisms related to *Bacillus subtilis*; identification and localization of *Bacillus subtilis* sequences of interest; evolutionary studies; determination of PanB protein regions required for function; modulation of a PanB protein activity or function; modulation of the activity of a pantothenate pathway; and modulation of cellular production of pantothenate.

The present invention provides a process for the production of pantothenate by fermentation. In particular, the present invention provides a process for production of pantothenate comprising converting a substrate into pantothenate. The substrate may be a carbon, nitrogen and phosphate source in which microorganisms are known to utilize to produce biomass, energy, and metabolites, such as pantothenate. A preferred substrate is α-ketoisovalerate. Suitable media as well as culture conditions for pantothenate production using *e*.*g. Bacillus subtilis* are known in the art and described *e*.*g*. in WO 2004113510A2 or WO 2007065602A1.

In connection with the above process using a microorganism, in one aspect, the process of the present invention leads to yields of pantothenate which are in general at least about more than at least about 10 to100% by using the modified PanB.

In one preferred embodiment, the present invention is related to a process for the production of pantothenate wherein a nucleotide according to the invention or a modified polynucleotide sequence as described above is introduced into a suitable microorganism as described herein, the recombinant microorganism is cultured under conditions that allow the production of pantothenate in high productivity, yield, and/or efficiency, the produced fermentation product is isolated from the culture medium and optionally further purified.

The recombinant microorganism carrying *e*.*g*. a modified *panB* gene and which is able to produce the fermentation product in significantly higher yield, productivity, and/or efficiency may be cultured in an aqueous medium supplemented with appropriate nutrients under aerobic conditions as described above.

The terms "production" or "productivity" are art-recognized and include the concentration of pantothenate formed within a given time and a given fermentation volume (*e*.*g*., kg product per hour per liter). The term "efficiency of production" includes the time required for a particular level of production to be achieved (for example, how long it takes for the cell to attain a particular rate of output of pantothenate). The term "yield" is art-recognized and includes the efficiency of the conversion of the carbon source into the product (*i*.*e*., pantothenate). This is generally written as, for example, kg product per kg carbon source. By "increasing the yield and/or production/productivity" of the compound it is meant that the quantity of recovered molecules, or of useful recovered molecules of that compound in a given amount of culture over a given amount of time is increased. The terms "biosynthesis" or a "biosynthetic pathway" are art-recognized and include the synthesis of a compound, preferably an organic compound, by a cell from intermediate compounds in what may be a multistep and highly regulated process.

Advantageous embodiments of the invention become evident from the dependent claims. These and other aspects and embodiments of the present invention should be apparent to those skilled in the art from the teachings herein.

This invention is further illustrated by the following examples which should not be construed as limiting. The contents of all references, patent applications, patents and published patent applications, cited throughout this application are hereby incorporated by reference, in particular WO 01/21772, WO 02/057474, WO 02/061108, WO 2004113510A2 or WO 2007065602A1.

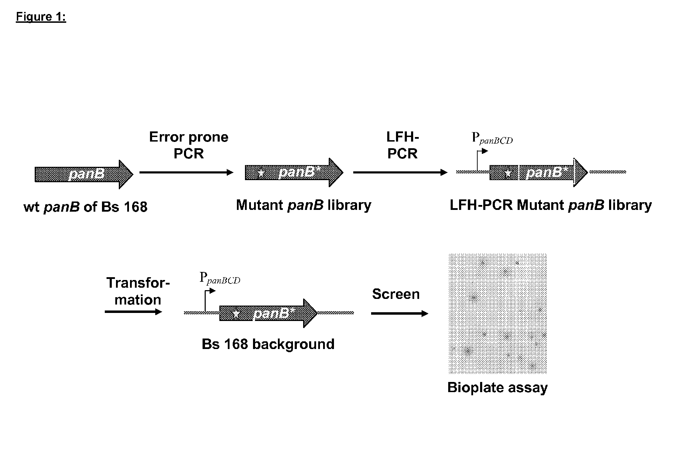

Figure 1: the different steps for constructing a genetically altered microorganism carrying modified *panB* gene downstream of the respective promoter via LFH-PCR followed by *in vivo* screen for PanB with enhanced pantothenate production is exemplified for the pantothenate (pan) operon comprising the genes *panB, panC, panD* (*panBCD*). Mutant genes are marked with a star ("*"). For more details see WO 2004113510A2.

### Examples

As otherwise indicated all strains, culture media as well as fermentation conditions are known in the art and described in more detail in WO 2004113510A2 or WO 2007065602A1 or W02004111214A1.

### Example 1: Generation of PanB mutant strains originating from B. subtilis 168 and measurement of pantothenate production

Error prone PCR was used to generate *B. subtilis* strains containing mutated *panB* either with the natural promoter or with the constitutive P15 promoter (Fig. 1). The following constructs were generated: M38I+N76S, G57C+L147M, D109E, G35E, 1244V, Q259H, G246R+G272S, and T226P. Furthermore, constructs containing 3 mutations, i.e. S13N+P75Q+A101V (SEQ ID NO:7), has been generated according to the protocol.

PanB mutants which contained the specific mutations were confirmed via sequencing. Furthermore, strains were generated wherein mutations S13N+P75Q+A101V were combined with a mutated *panE* gene together with the strong promoter P15 (see Example 1 of WO 2004113510A2).

Shake flask assay was performed as described before (W02004113510A2) wherein the newly constructed strains transformed with the constructs were grown in VFB MMGT supplemented with 1mM β-alanine. Samples were taken after 18 hours growth and analyzed by HPLC as described before (W02004113510A2). The results are shown in Table 1.

**Table 1: pantothenate production using modified PanB in comparison to the wild type B. subtilis 168. The designation of the mutation is originated from the amino acids which are exchanged and the position corresponding to a position on SEQ ID NO:2.**

| Mutation in PanB | Increase in pantothenate [fold] |
|---|---|
| PanB-wild type | 1 |
| M38I + N76S | 1.5 |
| G57C+L147M | 1.7 |
| D109E | 1.9 |
| G35E | 1.9 |
| 1244V | 1.9 |
| Q259H | 2.0 |
| S13N + P75Q + A101V | 1.9 |
| G246R + G272S | 3.1 |
| T226P | 5.2 |

A mutant strain carrying substitution 1244V was able to produce about 2-fold the amount of pantothenate in comparison to the wild-type strain, i.e. *B. subtilis* 168. An increase of more than about five times of the pantothenate production could be achieved with the mutation T226P.

Introduction of two PanB derivatives with mutation 1244V or the triple mutation (S13N + P75Q + A101V) into a pantothenate overexpression strain background wherein the natural promoters of the *panBCD* operon and the *panE* gene were replaced by the P15 strong promoter, respectively, resulted in ca. 11% increase in pantothenate production compared to the control pantothenate overexpression strain containing the wild-type, non-mutated *panB* gene.

## Claims

1. A modified polynucleotide selected from the group consisting of:
(a) polynucleotides encoding the polypeptide comprising the amino acid sequence according to SEQ ID NO:2;
(b) polynucleotides comprising the nucleotide sequence according to SEQ ID NO:1;
(c) polynucleotides comprising a nucleotide sequence obtainable by nucleic acid amplification such as polymerase chain reaction, using genomic DNA from a microorganism as a template and a primer set according to SEQ ID NO:3 and SEQ ID NO:4;
(d) polynucleotides comprising a nucleotide sequence encoding a fragment or derivative of a polypeptide encoded by a polynucleotide of any of (a) to (c) wherein in said derivative one or more amino acid residues are conservatively substituted compared to said polypeptide, and said fragment or derivative has ketopantoate hydroxymethyl transferase activity;
(e) polynucleotides the complementary strand of which hybridizes under stringent conditions to a polynucleotide as defined in any one of (a) to (d) and which encode a ketopantoate hydroxymethyl transferase polypeptide; and
(f) polynucleotides which are at least 60%, such as 70, 85, 90 or 95% identical to a polynucleotide as defined in any one of (a) to (d) and which encode a ketopantoate hydroxymethyl transferase polypeptide;
or
the complementary strand of such a polynucleotide and wherein the polynucleotide depicted under SEQ ID NO:1 comprises at least 1 mutation leading to an enzyme which is less sensitive to feedback inhibition.

2. A polynucleotide according to claim 1 said polynucleotide being selected from the group consisting of:
(a) polynucleotides encoding a (modified) polypeptide comprising the amino acid sequence according to SEQ ID NO:6, 8 or 10;
(b) polynucleotides comprising the (modified) nucleotide sequence according to SEQ ID NO:5, 7 or 9;
(c) polynucleotides comprising a nucleotide sequence obtainable by nucleic acid amplification such as polymerase chain reaction, using genomic DNA from a microorganism as a template and a primer set according to SEQ ID NO:3 and SEQ ID NO:4;
(d) polynucleotides comprising a nucleotide sequence encoding a fragment or derivative of a polypeptide encoded by a polynucleotide of any of (a) to (c) wherein in said derivative one or more amino acid residues are conservatively substituted compared to said polypeptide, and said fragment or derivative has the activity of modified ketopantoate hydroxymethyl transferase;
(e) polynucleotides the complementary strand of which hybridizes under stringent conditions to a polynucleotide as defined in any one of (a) to (d) and which encode a ketopantoate hydroxymethyl transferase polypeptide; and
(f) polynucleotides which are at least 60%, such as 70, 85, 90 or 95% identical to a polynucleotide as defined in any one of (a) to (d) and which encode a ketopantoate hydroxymethyl transferase polypeptide;
or
the complementary strand of such a polynucleotide and wherein the sequences depicted under SEQ ID NOs:5 to 10 are referred to as modified or mutated sequences and wherein said polynucleotide comprises at least one mutation leading to an enzyme which is less-sensitive to feedback inhibition compared to the corresponding wild-type polynucleotide.

3. A polynucleotide according to any one of claims 1 to 3 wherein the at least one mutation leads to increased production of pantothenate when introduced into a microorganism compared to the corresponding microorganism carrying the wild-type polynucleotide.

4. A polypeptide encoded by a polynucleotide according to any one of claims 1 to 3.

5. A microorganism genetically engineered with a polynucleotide according to any one of claims 1 to 3.

6. A microorganism according to claim 5 wherein the yield and/or efficiency of pantothenate production is improved compared to the wild-type microorganism.

7. A microorganism according to claim 5 or 6 capable of producing at least 11% more pantothenate compared to production of pantothenate using a wild type microorganism.

8. A microorganism according to any one of claims 5 to 7 producing a polypeptide according to claim 4 with reduced sensitivity to feedback inhibition compared to the wild type microorganism.

9. A microorganism according to any one of claims 5 to 8 wherein said microorganism is further genetically engineered in the panE gene leading to improved yield and/or efficiency of pantothenate production compared to the corresponding non-genetically engineered microorganism.

10. A microorganism according to any one of claims 5 to 9 selected from the group consisting of *Bacillus, Corynebacterium, Lactobacillus, Lactococcus, Streptomyces, Escherichia coli*, and *Pseudomonas.*

11. Use of a polynucleotide according to any one of claims 1 to 3 or of a microorganism according to any one of claims 5 to 10 for the production of pantothenate.

12. Use of a polynucleotide according to any one of claims 1 to 3 for genetically engineering a microorganism capable of pantothenate production.

13. Process for the production of a microorganism according to any one of claims 5 to 10 comprising the steps of:
(a) providing a suitable microorganism capable of pantothenate production,
(b) genetically engineering said microorganism with a polynucleotide according to any one of claims 1 to 3.

14. Process for reducing feedback inhibition of PanB in a microorganism capable of pantothenate production comprising introducing into said microorganism a polynucleotide according to any one of claims 1 to 3.

15. Process for the production of pantothenate wherein a microorganism according to any one of claims 5 to 10 is incubated in an aqueous medium under conditions that allow the production of pantothenate from a given substrate and optionally isolating and/or purifying the produced pantothenate from the reaction mixture.


SEQUENCE LISTING

<110> DSM IP Assets B.V.

<120> Increased vitamin production

<130> 26931

<160> 10

<170> PatentIn version 3.3

<210> 1
<211> 834
<212> DNA
<213> Bacillus subtilis 168

<400> 1
atgaaaacaa aactggattt tctaaaaatg aaggagtctg aagaaccgat tgtcatgctg 60

accgcttatg attatccggc agctaaactt gctgaacaag cgggagttga catgatttta 120

gtcggtgatt cacttggaat ggtcgtcctc ggccttgatt caactgtcgg tgtgacagtt 180

gcggacatga tccatcatac aaaagccgtt aaaaggggtg cgccgaatac ctttattgtg 240

acagatatgc cgtttatgtc ttatcacctg tctaaggaag atacgctgaa aaatgcagcg 300

gctatcgttc aggaaagcgg agctgacgca ctgaagcttg agggcggaga aggcgtgttt 360

gaatccattc gcgcattgac gcttggaggc attccagtag tcagtcactt aggtttgaca 420

ccgcagtcag tcggcgtact gggcggctat aaagtacagg gcaaagacga acaaagcgcc 480

aaaaaattaa tagaagacag tataaaatgc gaagaagcag gagctatgat gcttgtgctg 540

gaatgtgtgc cggcagaact cacagccaaa attgccgaga cgctaagcat accggtcatt 600

ggaatcgggg ctggtgtgaa agcggacgga caagttctcg tttatcatga tattatcggc 660

cacggtgttg agagaacacc taaatttgta aagcaatata cgcgcattga tgaaaccatc 720

gaaacagcaa tcagcggata tgttcaggat gtaagacatc gtgctttccc tgaacaaaag    780

cattcctttc aaatgaacca gacagtgctt gacggcttgt acgggggaaa ataa         834

<210>  2
<211>  277
<212>  PRT
<213>  Bacillus subtilis 168

<400>  2

Met Lys Thr Lys Leu Asp Phe Leu Lys Met Lys Glu Ser Glu Glu Pro
1               5                   10                  15

Ile Val Met Leu Thr Ala Tyr Asp Tyr Pro Ala Ala Lys Leu Ala Glu
            20                  25                  30

Gln Ala Gly Val Asp Met Ile Leu Val Gly Asp Ser Leu Gly Met Val
        35                  40                  45

Val Leu Gly Leu Asp Ser Thr Val Gly Val Thr Val Ala Asp Met Ile
    50                  55                  60

His His Thr Lys Ala Val Lys Arg Gly Ala Pro Asn Thr Phe Ile Val
65                  70                  75                  80

Thr Asp Met Pro Phe Met Ser Tyr His Leu Ser Lys Glu Asp Thr Leu
                85                  90                  95

Lys Asn Ala Ala Ala Ile Val Gln Glu Ser Gly Ala Asp Ala Leu Lys
            100                 105                 110

Leu Glu Gly Gly Glu Gly Val Phe Glu Ser Ile Arg Ala Leu Thr Leu
        115                 120                 125

Gly Gly Ile Pro Val Val Ser His Leu Gly Leu Thr Pro Gln Ser Val
    130                 135                 140

Gly Val Leu Gly Gly Tyr Lys Val Gln Gly Lys Asp Glu Gln Ser Ala
145                 150                 155                 160

Lys Lys Leu Ile Glu Asp Ser Ile Lys Cys Glu Glu Ala Gly Ala Met
                165                 170                 175

Met Leu Val Leu Glu Cys Val Pro Ala Glu Leu Thr Ala Lys Ile Ala
            180                 185                 190

Glu Thr Leu Ser Ile Pro Val Ile Gly Ile Gly Ala Gly Val Lys Ala
        195                 200                 205

Asp Gly Gln Val Leu Val Tyr His Asp Ile Ile Gly His Gly Val Glu
    210                 215                 220

Arg Thr Pro Lys Phe Val Lys Gln Tyr Thr Arg Ile Asp Glu Thr Ile
225                 230                 235                 240

Glu Thr Ala Ile Ser Gly Tyr Val Gln Asp Val Arg His Arg Ala Phe
                245                 250                 255

Pro Glu Gln Lys His Ser Phe Gln Met Asn Gln Thr Val Leu Asp Gly
            260                 265                 270

Leu Tyr Gly Gly Lys
        275

<210> 3
<211> 27

<212>  DNA
<213>  Artificial

<220>
<223>  primer

<400>  3
atgaaaacaa aactggattt tctaaaa                                    27


<210>  4
<211>  24
<212>  DNA
<213>  Artificial

<220>
<223>  primer

<400>  4
   ttttcccccg tacaagccgt caag                                    24


<210>  5
<211>  834
<212>  DNA
<213>  Bacillus subtilis

<400>  5
atgaaaacaa aactggattt tctaaaaatg aaggagtctg aagaaccgat tgtcatgctg     60

accgcttatg attatccggc agctaaactt gctgaacaag cgggagttga catgatttta    120

gtcggtgatt cacttggaat ggtcgtcctc ggccttgatt caactgtcgg tgtgacagtt    180

gcggacatga tccatcatac aaaagccgtt aaaaggggtg cgccgaatac ctttattgtg    240

acagatatgc cgtttatgtc ttatcacctg tctaaggaag atacgctgaa aaatgcagcg    300

gctatcgttc aggaaagcgg agctgacgca ctgaagcttg agggcggaga aggcgtgttt    360

gaatccattc gcgcattgac gcttggaggc attccagtag tcagtcactt aggtttgaca    420

ccgcagtcag tcggcgtact gggcggctat aaagtacagg gcaaagacga acaaagcgcc    480

aaaaaattaa tagaagacag tataaaatgc gaagaagcag gagctatgat gcttgtgctg    540

gaatgtgtgc cggcagaact cacagccaaa attgccgaga cgctaagcat accggtcatt    600

ggaatcgggg ctggtgtgaa agcggacgga caagttctcg tttatcatga tattatcggc    660

cacggtgttg agagaacacc taaatttgta aagcaatata cgcgcattga tgaaaccatc    720

gaaacagcag tcagcggata tgttcaggat gtaagacatc gtgctttccc tgaacaaaag    780

cattcctttc aaatgaacca gacagtgctt gacggcttgt acgggggaaa ataa          834

<210>  6
<211>  277
<212>  PRT
<213>  Bacillus subtilis

<400>  6

Met Lys Thr Lys Leu Asp Phe Leu Lys Met Lys Glu Ser Glu Glu Pro
1               5                   10                  15

Ile Val Met Leu Thr Ala Tyr Asp Tyr Pro Ala Ala Lys Leu Ala Glu
            20                  25                  30

Gln Ala Gly Val Asp Met Ile Leu Val Gly Asp Ser Leu Gly Met Val
        35                  40                  45

Val Leu Gly Leu Asp Ser Thr Val Gly Val Thr Val Ala Asp Met Ile
    50                  55                  60

His His Thr Lys Ala Val Lys Arg Gly Ala Pro Asn Thr Phe Ile Val
65                  70                  75                  80

Thr Asp Met Pro Phe Met Ser Tyr His Leu Ser Lys Glu Asp Thr Leu
                85                  90                  95

Lys Asn Ala Ala Ala Ile Val Gln Glu Ser Gly Ala Asp Ala Leu Lys
            100                 105                 110

Leu Glu Gly Gly Glu Gly Val Phe Glu Ser Ile Arg Ala Leu Thr Leu
        115                 120                 125

Gly Gly Ile Pro Val Val Ser His Leu Gly Leu Thr Pro Gln Ser Val
    130                 135                 140

Gly Val Leu Gly Gly Tyr Lys Val Gln Gly Lys Asp Glu Gln Ser Ala
145                 150                 155                 160

Lys Lys Leu Ile Glu Asp Ser Ile Lys Cys Glu Glu Ala Gly Ala Met
                165                 170                 175

Met Leu Val Leu Glu Cys Val Pro Ala Glu Leu Thr Ala Lys Ile Ala
            180                 185                 190

Glu Thr Leu Ser Ile Pro Val Ile Gly Ile Gly Ala Gly Val Lys Ala
        195                 200                 205

Asp Gly Gln Val Leu Val Tyr His Asp Ile Ile Gly His Gly Val Glu
    210                 215                 220

Arg Thr Pro Lys Phe Val Lys Gln Tyr Thr Arg Ile Asp Glu Thr Ile
225                 230                 235                 240

Glu Thr Ala Val Ser Gly Tyr Val Gln Asp Val Arg His Arg Ala Phe
                245                 250                 255

Pro Glu Gln Lys His Ser Phe Gln Met Asn Gln Thr Val Leu Asp Gly

260                 265                 270

Leu Tyr Gly Gly Lys
        275

<210>  7
<211>  834
<212>  DNA
<213>  Bacillus subtilis

<400>  7
atgaaaacaa aactggattt tctaaaaatg aaggagaatg aagaaccgat tgtgatgctg      60

accgcttatg attatccggc agctaagctt gctgaacaag cgggagttga catgatttta     120

gtcggtgatt cactcggaat ggtcgtcctc ggccttgatt caactgtcgg tgtgacagtt     180

gcggacatga tccatcatac aaaagctgtt aaaaggggcg cgcagaatac ttttattgtg     240

acagacatgc cgtttatgtc ttatcacctg tctaaggaag atacgctgaa aaatgcagca     300

gttatcgttc aggaaagcgg agctgacgca ctgaagcttg agggcggaga aggcgtgttt     360

gaatccattc gcgcattgac gcttggaggc attccagtag tcagtcactt aggtttgaca     420

ccgcagtcag tcggcgtact gggcggctat aaagtacagg gcaaagacga acaaagcgcc     480

aaaaaattaa tagaagacag tataaaatgc gaagaagcag gagctatgat gcttgtgctg     540

gaatgtgtgc cggcagaact cacagccaaa attgccgaga cgctaagcat accggtcatt     600

ggaatcgggg ctggtgtgaa agcggacgga caagttctcg tttatcatga tattatcggc     660

cacggtgttg agagaacacc taaatttgta aagcaatata cgcgcattga tgaaaccatc     720

gaaacagcaa tcagcggata tgttcaggat gtaagacatc gtgctttccc tgaacaaaag     780

cattcctttc aaatgaacca gacagtgctt gacggcttgt acgggggaaa ataa           834

<210>  8

<211>  277
<212>  PRT
<213>  Bacillus subtilis

<400>  8

Met Lys Thr Lys Leu Asp Phe Leu Lys Met Lys Glu Asn Glu Glu Pro
1               5                   10                  15

Ile Val Met Leu Thr Ala Tyr Asp Tyr Pro Ala Ala Lys Leu Ala Glu
            20                  25                  30

Gln Ala Gly Val Asp Met Ile Leu Val Gly Asp Ser Leu Gly Met Val
        35                  40                  45

Val Leu Gly Leu Asp Ser Thr Val Gly Val Thr Val Ala Asp Met Ile
    50                  55                  60

His His Thr Lys Ala Val Lys Arg Gly Ala Gln Asn Thr Phe Ile Val
65                  70                  75                  80

Thr Asp Met Pro Phe Met Ser Tyr His Leu Ser Lys Glu Asp Thr Leu
                85                  90                  95

Lys Asn Ala Ala Val Ile Val Gln Glu Ser Gly Ala Asp Ala Leu Lys
            100                 105                 110

Leu Glu Gly Gly Glu Gly Val Phe Glu Ser Ile Arg Ala Leu Thr Leu
        115                 120                 125

Gly Gly Ile Pro Val Val Ser His Leu Gly Leu Thr Pro Gln Ser Val
    130                 135                 140

Gly Val Leu Gly Gly Tyr Lys Val Gln Gly Lys Asp Glu Gln Ser Ala

145                 150                 155                 160

Lys Lys Leu Ile Glu Asp Ser Ile Lys Cys Glu Glu Ala Gly Ala Met
            165                 170                 175

Met Leu Val Leu Glu Cys Val Pro Ala Glu Leu Thr Ala Lys Ile Ala
        180                 185                 190

Glu Thr Leu Ser Ile Pro Val Ile Gly Ile Gly Ala Gly Val Lys Ala
    195                 200                 205

Asp Gly Gln Val Leu Val Tyr His Asp Ile Ile Gly His Gly Val Glu
210                 215                 220

Arg Thr Pro Lys Phe Val Lys Gln Tyr Thr Arg Ile Asp Glu Thr Ile
225                 230                 235                 240

Glu Thr Ala Ile Ser Gly Tyr Val Gln Asp Val Arg His Arg Ala Phe
            245                 250                 255

Pro Glu Gln Lys His Ser Phe Gln Met Asn Gln Thr Val Leu Asp Gly
        260                 265                 270

Leu Tyr Gly Gly Lys
    275

<210>  9
<211>  834
<212>  DNA
<213>  Bacillus subtilis

<400>  9
atgaaaacaa aactggattt tctaaaaatg aaggagaatg aagaaccgat tgtgatgctg      60

accgcttatg attatccggc agctaagctt gctgaacaag cgggagttga catgatttta 120

gtcggtgatt cactcggaat ggtcgtcctc ggccttgatt caactgtcgg tgtgacagtt 180

gcggacatga tccatcatac aaaagctgtt aaaaggggcg cgcagaatac ttttattgtg 240

acagacatgc cgtttatgtc ttatcacctg tctaaggaag atacgctgaa aaatgcagca 300

gttatcgttc aggaaagcgg agctgacgca ctgaagcttg agggcggaga aggcgtgttt 360

gaatccattc gcgcattgac gcttggaggc attccagtag tcagtcactt aggtttgaca 420

ccgcagtcag tcggcgtact gggcggctat aaagtacagg gcaaagacga acaaagcgcc 480

aaaaaattaa tagaagacag tataaaatgc gaagaagcag gagctatgat gcttgtgctg 540

gaatgtgtgc cggcagaact cacagccaaa attgccgaga cgctaagcat accggtcatt 600

ggaatcgggg ctggtgtgaa agcggacgga caagttctcg tttatcatga tattatcggc 660

cacggtgttg agagaacacc taaatttgta aagcaatata cgcgcattga tgaaaccatc 720

gaaacagcag tcagcggata tgttcaggat gtaagacatc gtgctttccc tgaacaaaag 780

cattcctttc aaatgaacca gacagtgctt gacggcttgt acgggggaaa ataa 834

<210> 10
<211> 277
<212> PRT
<213> Bacillus subtilis

<400> 10

Met Lys Thr Lys Leu Asp Phe Leu Lys Met Lys Glu Asn Glu Glu Pro
1               5                   10                  15

Ile Val Met Leu Thr Ala Tyr Asp Tyr Pro Ala Ala Lys Leu Ala Glu
            20                  25                  30

Gln Ala Gly Val Asp Met Ile Leu Val Gly Asp Ser Leu Gly Met Val

        35                  40                  45

Val Leu Gly Leu Asp Ser Thr Val Gly Val Thr Val Ala Asp Met Ile
    50                  55                  60

His His Thr Lys Ala Val Lys Arg Gly Ala Gln Asn Thr Phe Ile Val
65                  70                  75                  80

Thr Asp Met Pro Phe Met Ser Tyr His Leu Ser Lys Glu Asp Thr Leu
                85                  90                  95

Lys Asn Ala Ala Val Ile Val Gln Glu Ser Gly Ala Asp Ala Leu Lys
            100                 105                 110

Leu Glu Gly Gly Glu Gly Val Phe Glu Ser Ile Arg Ala Leu Thr Leu
        115                 120                 125

Gly Gly Ile Pro Val Val Ser His Leu Gly Leu Thr Pro Gln Ser Val
    130                 135                 140

Gly Val Leu Gly Gly Tyr Lys Val Gln Gly Lys Asp Glu Gln Ser Ala
145                 150                 155                 160

Lys Lys Leu Ile Glu Asp Ser Ile Lys Cys Glu Glu Ala Gly Ala Met
                165                 170                 175

Met Leu Val Leu Glu Cys Val Pro Ala Glu Leu Thr Ala Lys Ile Ala
            180                 185                 190

Glu Thr Leu Ser Ile Pro Val Ile Gly Ile Gly Ala Gly Val Lys Ala
        195                 200                 205

Asp Gly Gln Val Leu Val Tyr His Asp Ile Ile Gly His Gly Val Glu
    210                 215                 220

Arg Thr Pro Lys Phe Val Lys Gln Tyr Thr Arg Ile Asp Glu Thr Ile
225                 230                 235                 240

Glu Thr Ala Val Ser Gly Tyr Val Gln Asp Val Arg His Arg Ala Phe
                245                 250                 255

Pro Glu Gln Lys His Ser Phe Gln Met Asn Gln Thr Val Leu Asp Gly
            260                 265                 270

Leu Tyr Gly Gly Lys
        275